# EUROPÄISCHE PATENTSCHRIFT

(11) **EP 2 311 785 B1**
(45) Veröffentlichungstag und Bekanntmachung des Hinweises auf die Patenterteilung: **28.09.2011**
(21) Anmeldenummer: 09014089.8
(22) Anmeldetag: 11.11.2009
(51) Int. Cl.: C05F 17/00

(54) **Verfahren zur Behandlung von Abfällen**
Method for treating waste
Procédé de traitement de déchets

(30) Priorität: 16.10.2009 DE 102009049797
(43) Veröffentlichungstag der Anmeldung: 20.04.2011
(73) Patentinhaber: KOMPOFERM GmbH, 33428 Marienfeld (DE)
(72) Erfinder: Eggersmann, Karlgünter, 33428 Marienfeld (DE)
(74) Vertreter: Schober, Mirko

(56) Entgegenhaltungen:
- WO-A2-01/38259
- DE-A1- 4 417 248
- DE-C1- 19 602 023

## Beschreibung

Die Erfindung betrifft ein Verfahren zur Behandlung von Abfällen, insbesondere von Haus- oder Restmüll.

Bei der oben genannten Abfallbehandlung erfolgt üblicherweise eine Trennung des Abfalls in einzelne Fraktionen, zum Beispiel in im Wesentlichen organisches Material, Kunststoff, Metall und andere Abfallprodukte. Ziel ist es, den Abfall deponierbar zu machen oder auch zur thermischen Verwertung aufzubereiten. Um ein Deponat zu erhalten, muss insbesondere einem organischen Anteil des Abfalls die biologische Aktivität entzogen werden. Dies geschieht in der Regel durch eine aerobe Behandlung oder eine Kombination aus aerober und anaerober Behandlung, wobei das organikreiche Material volumetrisch getrennt wird.

Die aerobe Behandlung ist sehr aufwändig und energieintensiv und die angesprochene kombinierte Behandlung ist nur in einer sehr begrenzten Stückigkeit des organikreichen Materials mit Teilchengrößen kleiner 60 mm technisch realisierbar.

Dokument DE 4 417 248 A beschreibt ein Verfahren und eine Vorrichtung zum biologischen Abban organischer Abfälle durch Verknüpfung mehrerer Prozessketten mit einer Trennvorrichtung in eine Feinfraktion und in eine Grobfraktion für eine Weiterbehandlung in einem aeroben Verrottungsprozess.

Der Erfindung liegt daher die Aufgabe zugrunde, ein Verfahren zur Behandlung von Abfällen anzugeben, welches in Bezug auf die oben genannten Nachteile optimiert ist.

Gelöst wird diese Aufgabe durch ein Verfahren mit den Merkmalen des Anspruchs 1, vorteilhafte Ausführungsformen finden sich in den Unteransprüchen.

Erfindungsgemäß ist vorgesehen, aus dem Abfall eine organikreiche Fraktion abzutrennen, welche in einem anschließenden Schritt wiederum aufgeteilt wird in eine organikreiche Feinfraktion und eine organikreiche Grobfraktion. Die Feinfraktion weist bevorzugt Teilchen mit einer mittleren Korngröße zwischen 0 und 45 mm auf, die Grobfraktion weist bevorzugt Teilchen mit einer mittleren Korngröße von 10 bis 120 mm auf. Die Grobfraktion wird anschließend als Substrat einem Trockenfermentationsverfahren zugeführt und dabei zu einem Gärrest vergoren. Die Feinfraktion wird zusammen mit diesem Gärrest aerob behandelt und/oder getrocknet.

Diese Vorgehensweise hat den Vorteil, dass mittels der Trockenfermentation einem Teil der organikreichen Fracht unter Gewinnung von wiederverwertbarem Biogas Energie entzogen werden kann, die bei konventioneller aerober Behandlung verloren ginge. Zudem können bei der Trockenfermentation im Rahmen der Auslegung und Größe der entsprechenden Anlage beliebige organikreiche Abfallteile mit nahezu beliebigen Korngrößen verarbeitet werden.

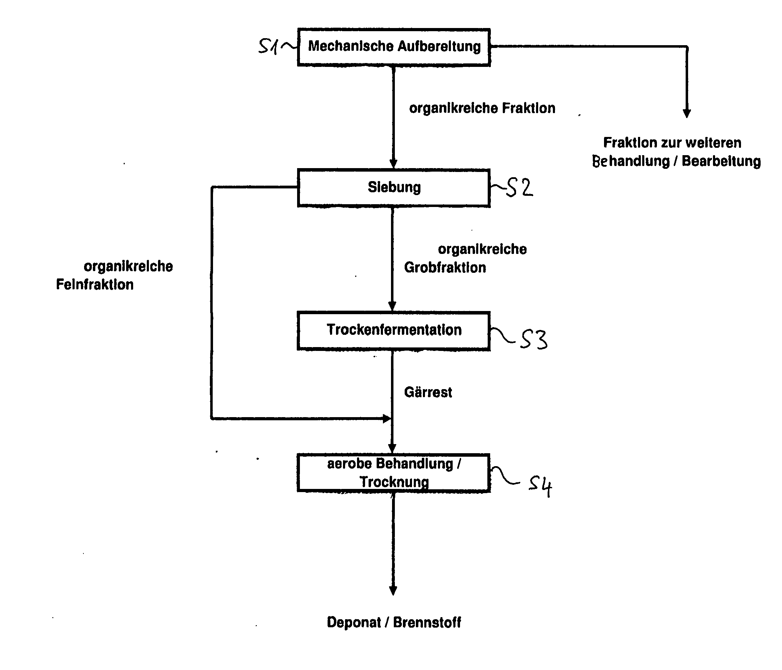

Die Erfindung wird nachfolgend anhand des Flussdiagramms in der einzigen Figur schematisch näher erläutert.

Das gezeigte Flussdiagramm stellt den erfindungsgemäßen Prozess vereinfacht dar. In einem ersten Schritt S1 erfolgt eine mechanische Aufbereitung des Abfalls, wobei eine Trennung des Abfalls in wenigstens eine erste Fraktion sowie in wenigstens eine organikreiche Fraktion erfolgt. Organikreich bedeutet, dass diese Fraktion jedenfalls einen beachtlichen Teil an organischem Material beinhaltet. Schritt S1 kann auch eine Größenselektion nach der mittleren Abfallteil- bzw. Korngröße beinhalten, wobei bevorzugt die organikreiche Fraktion Teile mit mittleren Korngrößen von 0 bis 120 mm, bevorzugter 0 bis 80 mm enthält, wobei der jeweils höhere Wert als vorgegebene Abfallteilgröße bezeichnet wird.

In einem weiteren Schritt S2 erfolgt eine weitere Trennung, z.B. Siebung der organischen Fraktion. Die Trennung erfolgt so, dass eine organische Feinfraktion mit Teilen einer mittleren Korngröße von 0 bis 45 mm, bevorzugt kleiner 30 mm, idealerweise kleiner 10 mm, und eine organische Grobfraktion mit Teilen einer mittleren Korngröße jeweils gleich oder oberhalb der genannten Werte entsteht. Die entstandene Grobfraktion wird in einem nächsten Schritt S3 als Substrat einem Trockenfermentationsverfahren unterzogen, welches als solches allgemein bekannt ist und hier nicht näher erläutert werden muss. Durch das Vergären der Grobfraktion im Trockenfermentationsschritt S3 entsteht ein Gärrest und gleichzeitig wird kontrolliert Biogas erzeugt, welches zur weiteren Verwendung aus dem Fermenter bzw. einem Perkolatbehälter entnommen werden kann. Dadurch wird der Grobfraktion Energie entzogen und gleichzeigt über Biogas für weitere Verwendungen zur Verfügung gestellt. Die Feinfraktion wird dann zusammen mit dem Gärrest einer weiteren Trocknung oder/und (aeroben) Behandlung zugeführt, so dass schließlich ein Deponat oder/und Brennstoff in nachgelagerten Behandlungsschritten S4 hergestellt werden kann.

## Patentansprüche

1. Verfahren zur Behandlung von Abfällen, bei welchem aus zugeführtem Abfallmaterial mittels nachgeschalteter Behandlungsschritte Deponat und/oder Brennstoff gewonnen wird und folgende Schritte aufweist:
a. Trennen des Abfallmaterials in wenigstens eine erste Fraktion, insbesondere mit Abfallteilen oberhalb einer vorgegebenen Abfallteilgröße, und in eine zweite organikreiche Fraktion, insbesondere mit Abfallteilen unterhalb der vorgegebenen Abfallteilgröße (S1),
b. Trennen der zweiten Fraktion in wenigstens eine organikreiche Feinfraktion und eine organikreiche Grobfraktion (S2),
c. Vergären der organikreichen Grobfraktion mittels Trockenfermentation (S3),
d. weitere Behandlung des Gärrestes aus der Trockenfermentation zusammen mit der zuvor abgesonderten organikreichen Feinfraktion (S4).

2. Verfahren nach Anspruch 1,
**dadurch gekennzeichnet,**
**dass** die weitere Behandlung nach Schritt d. eine aerobe Behandlung und/oder eine Trocknung umfasst.

3. Verfahren nach einem der vorherigen Ansprüche,
**dadurch gekennzeichnet,**
**dass** das Trennen gemäß Schritt b. durch Sieben erfolgt.

4. Verfahren nach einem der vorherigen Ansprüche,
**dadurch gekennzeichnet,**
**dass** die vorgegebene Abfallteilgröße im Mittel zwischen 60 und 120 mm liegt.

5. Verfahren nach Anspruch 4,
**dadurch gekennzeichnet,**
**dass** die vorgegebene Abfallteilgröße im Mittel etwa bei 80 mm liegt.

6. Verfahren nach einem der vorherigen Ansprüche,
**dadurch gekennzeichnet,**
**dass** die mittlere Größe der Abfallteile der organikreichen Feinfraktion kleiner als 10 bis 45 mm ist.

7. Verfahren nach Anspruch 6,
**dadurch gekennzeichnet,**
**dass** die mittlere Größe der Abfallteile der organikreichen Feinfraktion kleiner als 30 mm ist.

## Claims

1. Method for treating waste materials in which deposits and/or combustible material are extracted by means of a series of treatment steps from the waste material supplied and comprises the following steps:
a. Separating the waste material into at least one first fraction, more particularly with waste parts above a predetermined waste part size, and into a second organic-rich fraction, more particularly with waste parts below the predetermined waste part size (S1)
b. Separating the second fraction into at least one organic-rich fine fraction and into organic-rich coarse fraction (S2)
c. Fermenting the organic-rich coarse fraction by means of dry fermentation (S3)
d. Further treating the fermented residue from the dry fermentation together with the previously separated organic-rich fine fraction (S4).

2. Method according to claim 1
**characterised in that**
the further treatment according to step d. comprises an aerobic treatment and/or drying.

3. Method according to one of the preceding claims
**characterised in that**
the separation according to step b. takes place by screening.

4. Method according to one of the preceding claims
**characterised in that**
the predetermined waste part size lies on average between 60 and 120 mm.

5. Method according to claim 4
**characterised in that**
the predetermined waste part size lies on average at 80 mm.

6. Method according to one of the previous claims
**characterised in that**
the average size of the waste parts of the organic-rich fine fraction is smaller than 10 to 45 mm.

7. Method according to claim 6
**characterised in that**
the average size of the waste particles of the organic-rich fine-fraction is smaller than 30 mm.

## Revendications

1. Procédé de traitement de déchets, dans lequel une matière pouvant être mise en décharge et / ou du carburant est sont tirés des déchets, par des phases de traitement successives, et qui comprend les étapes suivantes:
a. Tri des déchets en au moins une fraction, qui comprend en particulier des déchets de taille supérieurs à une grandeur de déchets prédéterminée, et en une deuxième fraction riche en matières organiques, qui comprend en particulier des déchets de taille au-dessous de la grandeur de déchets prédéterminée (S1),
b. Séparation de la deuxième fraction en au moins une fraction fine riche en matières organiques et une fraction grossière riche en matières organiques (S2),
c. Fermentation de la fraction grossière riche en matières organiques par fermentation à sec (S3),
d. Traitement ultérieur du résidu de fermentation de la fermentation à sec, en commun avec la fraction fine riche en matières organiques (S4) précédemment séparée.

2. Procédé selon la revendication 1,
**caractérisé en ce que**
le traitement faisant selon l'étape d. comprend un traitement aérobie et / ou un séchage.

3. Procédé selon l'une des revendications précédentes,
**caractérisé en ce que**
la séparation selon l'étape b. est effectuée par criblage.

4. Procédé selon l'une des revendications précédentes,
**caractérisé en ce que**
la grandeur de déchets prédéterminée est située entre 60 et 120 mm.

5. Procédé selon la revendication 4,
**caractérisé en ce que**
la grandeur de déchets prédéterminée est en moyenne de 80 mm.

6. Procédé selon l'une des revendications précédentes,
**caractérisé en ce que**
la grandeur moyenne des déchets de la fraction fine riche en matières organiques est inférieure à 10 à 45 mm.

7. Procédé selon la revendication 6,
**caractérisé en ce que**
la grandeur moyenne des déchets de la fraction fine riche en matières organiques est inférieure à 30 mm.
